# EUROPEAN PATENT SPECIFICATION

(11) **EP 2 510 890 B1**
(45) Date of publication and mention of the grant of the patent: **03.08.2022**
(21) Application number: 12163881.1
(22) Date of filing: 12.04.2012
(51) Int. Cl.: A61B 18/14

(54) **Surgical forceps and method of manufacturing thereof**
Chirurgische Zange und Herstellungsverfahren dafür
Pince chirurgicale et son procédé de fabrication

(30) Priority: 12.04.2011 US 201113085144
(43) Date of publication of application: 17.10.2012
(73) Proprietor: Covidien LP, Mansfield, MA 02048 (US)
(72) Inventor: Hart, Keir, Lafayette, CO, 80026 (US); Hempstead, Russell, Lafayette, CO, 80026 (US)
(74) Representative: Soames, Candida Jane

(56) References cited:
- EP-A1- 1 707 143
- WO-A2-2007/047380
- US-A- 5 649 937
- US-A1- 2002 120 267
- US-A1- 2009 082 769

## Description

### BACKGROUND

The present invention relates to methods of manufacturing surgical forceps.

### Technical Field

A forceps is a plier-like instrument which relies on mechanical action between its jaws to grasp, clamp and constrict vessels or tissue. Electrosurgical forceps utilize both mechanical clamping action and electrical energy to affect hemostasis by heating tissue and blood vessels to coagulate and/or cauterize tissue. Certain surgical procedures require more than simply cauterizing tissue and rely on the unique combination of clamping pressure, precise electrosurgical energy control and gap distance (i.e., distance between opposing jaw members when closed about tissue) to "seal" tissue, vessels and certain vascular bundles. Typically, once a vessel is sealed, the surgeon has to accurately sever the vessel along the newly formed tissue seal. Accordingly, many vessel sealing instruments have been designed which incorporate a knife or blade member that effectively severs the tissue after forming a tissue seal.

US 2009/082769 A1 discloses a method for manufacturing an end effector assembly for sealing tissue including the initial step of providing a pair of first and second jaw members each having an outer insulative housing and an electrically conductive tissue sealing plate, respectively. The method also includes the steps of disposing a series of insulative stop members atop an insulative substrate of at least one of the jaw members and forming a corresponding series of apertures within the electrically conductive sealing plate of the jaw member in vertical registry with the stop members. The method further comprises the steps of: aligning the electrically conductive sealing plate of the jaw member atop the insulative substrate such that each of the series of stop members is received through a respective aperture within the electrically conductive sealing plate and securing the electrically conductive sealing plate atop the insulative substrate such that the stop members project from the electrically conductive sealing plate.

### SUMMARY

The present invention provides a method of making a forceps, wherein the forceps comprises: an end effector assembly including first and second jaw members, at least one of the jaw members movable relative to the other between a spaced-apart position and an approximated position for grasping tissue therebetween, the method comprising the following steps in the manufacture of at least one of the jaw members: providing an insulator including at least one engagement knob extending therefrom, the at least one engagement knob having a diameter "d"; providing a tissue-sealing plate (122) having at least one engagement aperture defined therethrough, the tissue sealing plate (122) defining a tissue-sealing surface; and positioning the tissue-sealing plate (122) about the insulator (126) such that the at least one engagement knob (126c) is inserted through the at least one engagement aperture (122b) and such that a free end (126d) of the at least one engagement knob (126c) extends through the at least one engagement aperture (122b) and extends from the tissue-sealing surface of the tissue-sealing plate (122), characterised in that the method further comprises deforming the free end (126d) of the at least one engagement knob (126c) such that the free end (126d) of the at least one engagement knob (126c) is deformed from a first configuration having diameter "d" which is less than a diameter of the at least one engagement aperture (122b), to a second configuration having a diameter "D" which is greater than the diameter of the at least one engagement aperture (122b), thereby preventing the at least one engagement knob (126c) from passing through the at least one engagement aperture (122b) and securing the insulator (126) and the sealing plate (122) to one another, wherein the step of deforming the free end (126d) of the at least one engagement knob (126c) is performed via heat-staking, such that in the second configuration, the free end (126d) of the at least one engagement knob (126c) protrudes a pre-determined distance from the tissue-sealing surface to set a gap distance between the jaw members (110, 120) when the jaw members (110, 120) are disposed in the approximated position.

In embodiments, the forceps may include a knife assembly. The knife assembly includes a knife bar and a knife blade. The knife bar is selectively translatable relative to the end effector assembly to translate the knife blade between a retracted position and an extended position, wherein the knife blade extends between the jaw members to cut tissue grasped therebetween. Further, the knife blade and the knife bar may be secured to one another via heat staking.

In embodiments, one or both of the jaw members includes a jaw frame and an insulator. The jaw frame includes one or more engagement apertures defined therethrough. The insulator includes one or more engagement knobs extending therefrom. The engagement knob is configured for insertion through the engagement aperture upon positioning of the insulator atop the jaw frame. A free end of the engagement knob is configured to extend through the engagement aperture and to extend from a surface of the jaw frame. The free end of the engagement knob is deformable from a first configuration, wherein the free end of the engagement knob is freely removable from the engagement aperture, to a second configuration, wherein the free end of the engagement knob is permanently inhibited from passing through the engagement aperture, thereby engaging the insulator and the jaw frame to one another.

In these embodiments, the free end of the engagement knob is suitably deformed from the first configuration to the second configuration via heat staking.

In these embodiments, the jaw frame may define a lip disposed about a periphery of the engagement aperture on an underside of the jaw frame. The lip is configured to receive the free end of the engagement knob therein when the engagement knob is disposed in the second configuration. Further, in the second configuration, the free end of the engagement knob may be disposed within the lip such that the free end of the engagement knob is substantially flush with the jaw frame along a surface thereof.

### BRIEF DESCRIPTION OF THE DRAWINGS

Various embodiments of the present invention are described herein with reference to the drawings wherein:
Fig. 1 is a front, perspective view of an endoscopic surgical forceps manufactured in accordance with the present invention;
Fig. 2 is a front, perspective view of an open surgical forceps manufactured in accordance with the present invention;
Fig. 3 is an enlarged, front, perspective view of an end effector assembly portion of the forceps of Figs. 1 and 2;
Fig. 4A is a transverse, cross-sectional view of a sealing plate and insulator configured for manufacture of a jaw member of the end effector assembly of Fig. 3, shown with parts separated;
Fig. 4B is a transverse, cross-sectional view of the sealing plate and insulator of Fig. 4A shown in position for assembly;
Fig. 4C is a transverse, cross-sectional view of the sealing plate and insulator of Fig. 4A shown in an assembled condition;
Fig. 5A is a side view of another jaw member configured for use with the end effector assembly of Fig. 3, shown with parts separated;
Fig. 5B is a bottom, perspective view of the jaw member of Fig. 5A, shown in position for assembly;
Fig. 5C is a bottom, perspective view of the jaw member of Fig. 5A, shown in an assembled condition;
Fig. 6A is a longitudinal, cross-sectional view of the end effector assembly of Fig. 3 with the jaw members disposed in a spaced-apart position;
Fig. 6B is a longitudinal, cross-sectional view of the end effector assembly of Fig. 3 with the jaw members disposed in an approximated position and with a knife blade disposed in a retracted position;
Fig. 6C is a longitudinal, cross-sectional view of the end effector assembly of Fig. 3 with the jaw members disposed in an approximated position and with the knife blade disposed in an extended position;
Fig. 7A is a longitudinal, cross-sectional view of a knife assembly configured for use with the end effector assembly of Fig. 3, shown with parts separated;
Fig. 7B is a longitudinal, cross-sectional view of the knife assembly Fig. 7A, shown in position for assembly; and
Fig. 7C is a longitudinal, cross-sectional view of the knife assembly Fig. 7A , shown in an assembled condition.

### DETAILED DESCRIPTION

Embodiments of the present invention are described in detail with reference to the drawing figures wherein like reference numerals identify similar or identical elements. As used herein, the term "distal" refers to the portion that is being described which is further from a user, while the term "proximal" refers to the portion that is being described which is closer to a user.

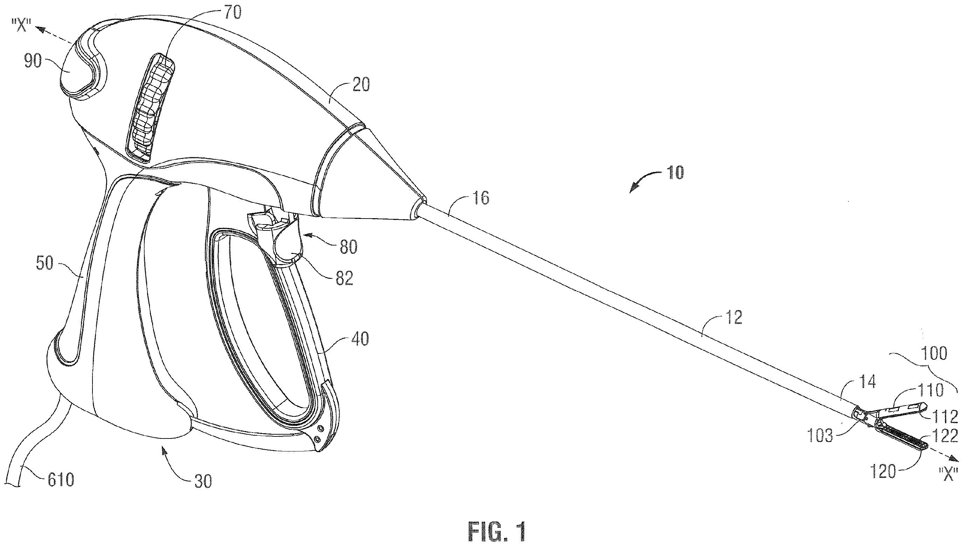

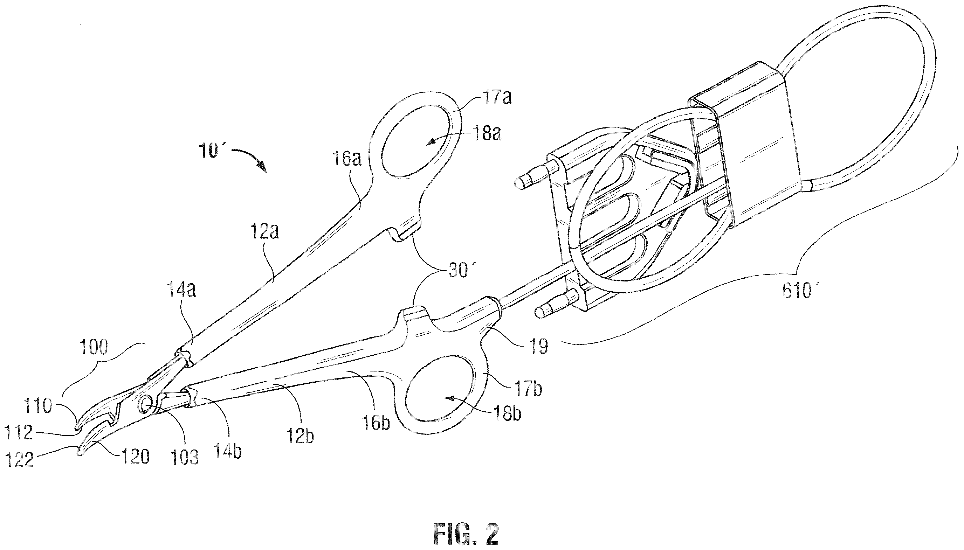

Referring now to Figs. 1 and 2, Fig. 1 depicts a forceps 10 for use in connection with endoscopic surgical procedures and Fig. 2 depicts an open forceps 10' contemplated for use in connection with traditional open surgical procedures. For the purposes herein, either an endoscopic instrument, e.g., forceps 10, or an open instrument, e.g., forceps 10', may be utilized in accordance with the present disclosure. Obviously, different electrical and mechanical connections and considerations apply to each particular type of instrument, however, the novel aspects with respect to the end effector assembly and its operating characteristics remain generally consistent with respect to both the open and endoscopic configurations.

Turning now to Fig. 1, an endoscopic forceps 10 is provided defining a longitudinal axis "X-X" and including a housing 20, a handle assembly 30, a rotating assembly 70, a trigger assembly 80 and an end effector assembly 100. Forceps 10 further includes a shaft 12 having a distal end 14 configured to mechanically engage end effector assembly 100 and a proximal end 16 that mechanically engages housing 20. Forceps 10 also includes electrosurgical cable 610 that connects forceps 10 to a generator (not shown) or other suitable power source, although forceps 10 may alternatively be configured as a battery powered instrument. Cable 610 includes a wire (or wires) (not shown) extending therethrough that has sufficient length to extend through shaft 12 in order to provide electrical energy to at least one of the sealing plates 112, 122 of jaw members 110, 120, respectively, of end effector assembly 100, e.g., upon activation of activation switch 90.

With continued reference to Fig. 1, handle assembly 30 includes fixed handle 50 and a moveable handle 40. Fixed handle 50 is integrally associated with housing 20 and handle 40 is moveable relative to fixed handle 50. Rotating assembly 70 is rotatable in either direction about longitudinal axis "X-X" to rotate end effector 100 about longitudinal axis "X-X." Housing 20 houses the internal working components of forceps 10.

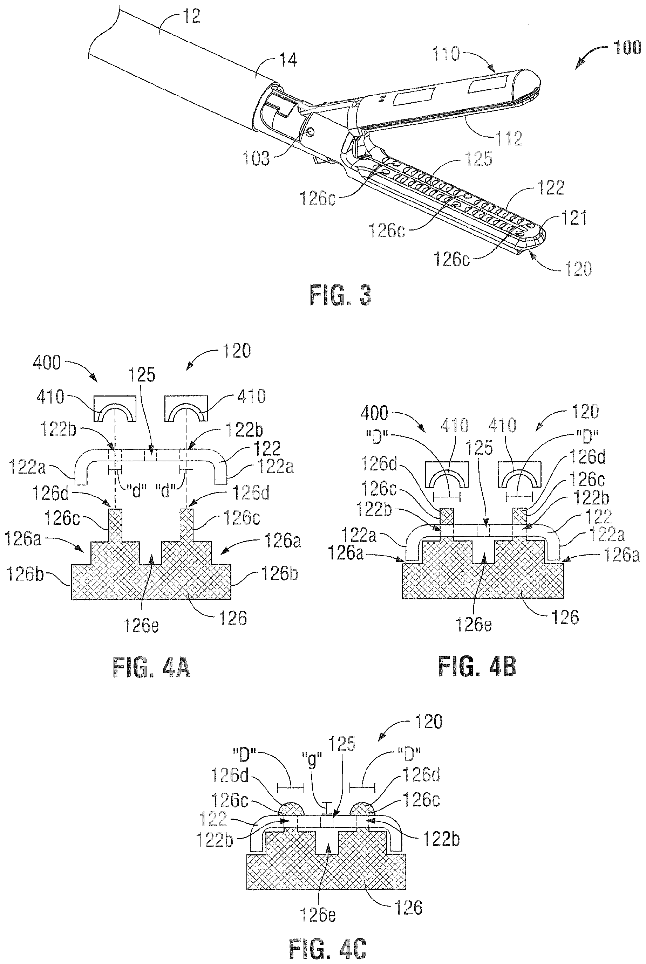

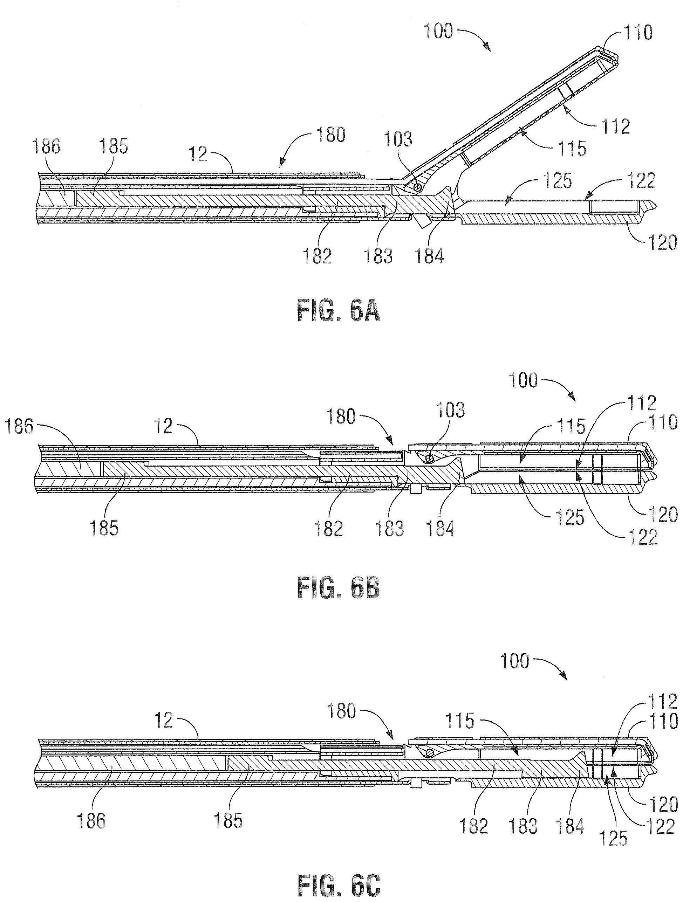

End effector assembly 100 is shown attached at a distal end 14 of shaft 12 and includes a pair of opposing jaw members 110 and 120. Each of the jaw members 110 and 120 includes an opposed electrically conductive tissue-sealing plate 112, 122, respectively. End effector assembly 100 is designed as a unilateral assembly, i.e., where jaw member 120 is fixed relative to shaft 12 and jaw member 110 is moveable about pivot 103 relative to shaft 12 and fixed jaw member 120. However, end effector assembly 100 may alternatively be configured as a bilateral assembly, i.e., where both jaw member 110 and jaw member 120 are moveable about a pivot 103 relative to one another and to shaft 12. In some embodiments, a knife assembly 180 (Figs. 6A-6C) is disposed within shaft 12 and a knife channel 125 (Fig. 3) is defined within one or both jaw members 110, 120 to permit reciprocation of a knife blade 182 (Fig. 6A-6C) therethrough, e.g., via activation of a trigger 82 of trigger assembly 80. The particular features of end effector assembly 100 will be described in greater detail hereinbelow.

Continuing with reference to Fig. 1, moveable handle 40 of handle assembly 30 is ultimately connected to a drive assembly (not shown) that, together, mechanically cooperate to impart movement of jaw members 110 and 120 between a spaced-apart position and an approximated position to grasp tissue disposed between sealing plates 112 and 122 of jaw members 110, 120, respectively. As shown in Fig. 1, moveable handle 40 is initially spaced-apart from fixed handle 50 and, correspondingly, jaw members 110, 120 are in the spaced-apart position. Moveable handle 40 is actuatable from this initial position to a depressed position corresponding to the approximated position of jaw members 110, 120 (see Figs. 6B-6C).

Referring now to Fig. 2, an open forceps 10' is shown including two elongated shafts 12a and 12b, each having a proximal end 16a and 16b, and a distal end 14a and 14b, respectively. Similar to forceps 10 (Fig. 1), forceps 10' is configured for use with end effector assembly 100. More specifically, end effector assembly 100 is attached to distal ends 14a and 14b of shafts 12a and 12b, respectively. As mentioned above, end effector assembly 100 includes a pair of opposing jaw members 110 and 120 that is pivotably connected about a pivot 103. Each shaft 12a and 12b includes a handle 17a and 17b disposed at the proximal end 16a and 16b thereof. Each handle 17a and 17b defines a finger hole 18a and 18b therethrough for receiving a finger of the user. As can be appreciated, finger holes 18a and 18b facilitate movement of the shafts 12a and 12b relative to one another which, in turn, pivots jaw members 110 and 120 from an open position, wherein the jaw members 110 and 120 are disposed in spaced-apart relation relative to one another, to a closed position, wherein the jaw members 110 and 120 cooperate to grasp tissue therebetween.

A ratchet 30' may be included for selectively locking the jaw members 110 and 120 relative to one another at various positions during pivoting. Ratchet 30' may include graduations or other visual markings that enable the user to easily and quickly ascertain and control the amount of closure force desired between the jaw members 110 and 120.

With continued reference to Fig. 2, one of the shafts, e.g., shaft 12b, includes a proximal shaft connector 19 that is designed to connect the forceps 10' to a source of electrosurgical energy such as an electrosurgical generator (not shown). Proximal shaft connector 19 secures an electrosurgical cable 610' to forceps 10' such that the user may selectively apply electrosurgical energy to the electrically conductive sealing plates 112 and 122 of jaw members 110 and 120, respectively, as needed.

Forceps 10' may further include a knife assembly 180 (Figs. 6A-6C) disposed within either of shafts 12a, 12b and a knife channel 115, 125 ( Fig. 6A) defined within one or both of jaw members 110, 120, respectively, to permit reciprocation of a knife blade 182 ( Figs. 6A-6C) therethrough.

Turning now to Fig. 3, end effector assembly 100, including jaw members 110 and 120 is configured for use with either forceps 10 or forceps 10', discussed above, or any other suitable surgical instrument capable of pivoting jaw members 110, 120 relative to one another between a spaced-apart position and an approximated position for grasping tissue therebetween. However, for purposes of simplicity and consistency, end effector assembly 100 will be described hereinbelow with reference to forceps 10 only. Further, jaw members 110, 120 are substantially similar to one another and, thus, only the description of jaw member 120 will be detailed below for the purposed of brevity.

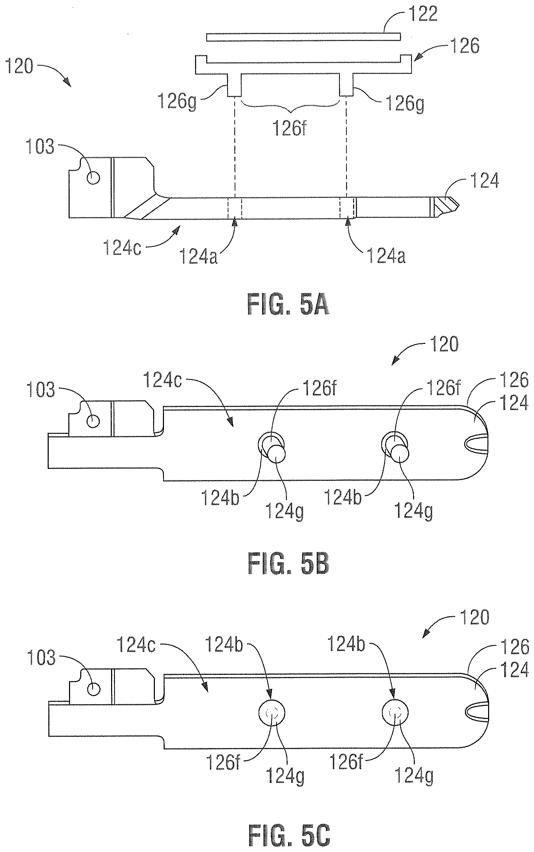

Jaw member 120, as shown in Fig. 3, includes an outer jaw housing 121 and an electrically-conductive tissue-sealing plate 122. Jaw member 120 further includes a jaw frame 124 (Figs. 5A-5C) and an insulator 126 (Figs. 4A-4C). Jaw frame 124 (Figs. 5A-5C) is coupled to the jaw frame (not shown) of jaw member 110 via pivot 103 to permit pivotable movement of jaw members 110, 120 between the spaced-apart and approximated positions and is configured to support the other components of jaw member 120 thereon. Insulator 126 (Figs. 4A-4C) is disposed on jaw frame 124 (Figs. 5A-5C) and is configured to retain tissue-sealing plate 122 thereon. Outer jaw housing 121 is disposed about jaw frame 124 (Figs. 5A-5C) and insulator 126 (Figs. 4A-4C) and is configured to retain the components of jaw member 120 therein, while tissue-sealing plate 122 defines an exposed tissue-sealing surface that opposes the tissue-sealing surface defined by tissue-sealing plate 112 of jaw member 110, as shown in Fig. 3 . Various methods and configurations for manufacturing an end effector assembly, e.g., end effector assembly 100, or the components thereof, are described in detail hereinbelow.

Referring now to Figs. 4A-4C, the configuration and manufacture of insulator 126 and sealing plate 122 of jaw member 120 in accordance with an embodiment of the present disclosure is described. Insulator 126 is made from an electrically-insulative material and, as mentioned above, is configured to retain sealing plate 122 thereon. Insulator 126 is further configured for positioning atop jaw frame 124 (Figs. 5A-5C) and may be secured thereto via any suitable method, e.g., heat-staking, as will be described in greater detail below. More particularly, as shown in Figs. 4A-4C, insulator 126 includes a pair of longitudinally-extending grooves 126a defined at opposed longitudinal sides 126b thereof. Grooves 126a are configured to receive wings 122a of sealing plate 122 therein upon positioning of sealing plate 122 about insulator 126. Insulator 126 further includes a pair of sealing plate engagement knobs 126c extending therefrom. As best shown in Fig. 3, insulator 126 may include several pairs of sealing plate engagement knobs 126c disposed along the length thereof, although insulator 126 may also include individual engagement knobs 126c disposed thereon or may include sealing plate engagement knobs 126c extending therefrom in any other suitable configuration. Sealing plate 122, on the other hand, includes a plurality of engagement apertures 122b defined therethrough, each of which is configured to receive one of the sealing plate engagement knobs 126c therein such that a free end 126d of each of the sealing plate engagement knobs 126c extends therefrom, as best shown in Fig. 4B. As shown in Figs 4A-4B, sealing plate engagement knobs 126c initially define a generally uniform, cylindrical configuration to facilitate passage through the generally circular engagement apertures 122b of sealing plate 122, although other complementary configurations may also be provided.

With continued reference to Figs. 4A-4C, the assembly of insulator 126 and sealing plate 122 is described. Initially, as shown in Figs. 4A-4B, sealing plate 122 is positioned atop insulator 126 such that wings 122a of sealing plate 122 are disposed within grooves 126a of insulator 126 and such that sealing plate engagement knobs 126c of insulator 126 extend through the respective engagement apertures 122b of sealing plate 122 with the free ends 126d thereof extending from engagement apertures 122b. As can be appreciated, a desired sealing plate 122 and insulator 126 may be selected, e.g., a sealing plate 122 and insulator 126 of a specific configuration, made from a particular material, etc., such that various different combinations may be formed. Insulator 126 may further include a knife channel 126e configured to align with knife channel 125 of sealing plate 122 upon positioning of sealing plate 122 thereon to permit reciprocation of a knife blade 182 (Figs. 6A-6C) therethrough.

Referring now to Fig. 4B, with wings 122a of sealing plate 122 disposed within grooves 126a of insulator 126 and with sealing plate engagement knobs 126c of insulator 126 extending through engagement apertures 122b of sealing plate 122, sealing plate 122 and insulator 126 are retained in fixed position relative to one another except that, at this point, sealing plate 122 may be easily separated from insulator 126, thus returning to the position shown in Fig. 4A.

In order to secure sealing plate 122 atop insulator 126, a heat-staking element 400 is used to deform free ends 126d of sealing plate engagement knobs 126c of insulator 126 such that sealing plate engagement knobs 126c are inhibited from being translated back through engagement apertures 122b, i.e., such that removal of sealing plate 122 from insulator 126 is inhibited. More specifically, heat-staking element 400 includes a head 410 that defines a dome-shaped configuration, although other configurations are contemplated. The dome-shaped head 410 of heat-staking element 400 defines a diameter "D" that is greater than the diameter "d" of engagement apertures 122b of sealing plate 122.

During assembly, the dome-shaped head 410 of heat-staking element 400 is positioned about free end 126d of each sealing plate engagement knob 126c and is heated to a sufficient temperature to permit deformation of sealing plate engagement knobs 126c of insulator 126, but such that sealing plate 122 remains substantially unaffected. As can be appreciated, the specific temperature may depend on the material(s) forming insulator 126 and/or seal plate 122. As free ends 126d of sealing plate engagement knobs 126c are heated and, ultimately, become deformable, free ends 126d of sealing plate engagement knobs 126c conform to the dome-shaped head 410 of heat-staking element 400, thereby defining a complementary dome-shaped configuration, as shown in Fig. 4C. Sealing plate engagement knobs 126c are then permitted to cool such that the free ends 126d of sealing plate engagement knobs 126c retain this dome-shaped deformed configuration. In this deformed configuration, free ends 126d of sealing plate engagement knobs 126c define a dome-shaped configuration having a diameter "D," similar to that of dome-shaped head 410 of heat-staking element 400. Since the deformed free ends 126d of sealing plate engagement knobs 126c define a diameter "D," that is greater than the diameter "d" of engagement apertures 122b of sealing plate 122, free ends 126d of sealing plate engagement knobs 126c are inhibited from passing back through engagement apertures 122b of sealing plate 122. In other words, in this position, sealing plate 122 is secured about insulator 126.

Referring now to Fig. 3 in conjunction with Fig. 4C, heat-staking element 400 (Figs. 4A-4B) may be configured such that sealing plate engagement knobs 126c, once deformed, extend a pre-determined distance "g" from the tissue-sealing surface of sealing plate 122. This pre-determined distance "g" defines a minimum gap distance "g" between the sealing surfaces of sealing plates 112, 122 of jaw members 110, 120, respectively, upon movement of jaw members 110, 120 to the approximated position. However, where each jaw member 110, 120 includes opposed sealing plate engagement knobs 126c extending therefrom, the heat-staking element 400 may be configured to deform the sealing plate engagement knobs 126c to extend half the distance "g" such that, upon approximation of jaw members 110, 120, the opposed sealing plate engagement knobs 126c cooperate to define the minimum gap distance "g" between sealing plates 112, 122 of jaw members 110, 120, respectively. As can be appreciated, depending on the procedure to be performed, the size and/or composition of tissue to be sealed, etc., the desired gap distance "g" may vary. Typically, for tissue-sealing, the gap distance "g" is in the range of 0.0254 mm to 0.1524 mm (0.001 inches to 0.006 inches).

Additionally, or alternatively, insulator 126, and/or sealing plate engagement knobs 126c thereof, may be formed from a resiliently compressible material to facilitate achieving a desired gap distance "g" between sealing plates 112, 122 of jaw members 110, 120, respectively, in accordance with the closure pressure between jaw members 110, 120 when jaw members 110, 120 are moved to the approximated position to grasp tissue therebetween, as will be described in greater detail below. Typically, the closure pressure between jaw members 110, 120 is in the range of about 3 kg/cm² to about 16 kg/cm².

Continuing with reference to Fig. 3, in use, with jaw members 110, 120 in the spaced-apart position, end effector assembly 100 is positioned such that tissue to be grasped, sealed and/or divided is disposed between sealing plates 112, 122 of jaw members 110, 120, respectively. Thereafter, jaw members 110, 120 are moved to the approximated position to grasp tissue between tissue-sealing plates 112 and 122, e.g., via depressing moveable handle 40 of forceps 10 from the initial position to the depressed position relative to fixed handle 50 (see Fig. 1).

Upon moving jaw members 110, 120 toward the approximated position to grasp tissue therebetween, the deformed free ends 126d of sealing plate engagement knobs 126c of jaw member 120 contact corresponding components of jaw member 110 (or simply contact sealing plate 112 of jaw member 110) to set the gap distance "g" between tissue-sealing plates 112, 122 of jaw members 110, 120, respectively. In embodiments where sealing plate engagement knobs 126c are resiliently compressible, upon approximation of jaw members 110, 120, sealing plate engagement knobs 126c are compressed between sealing plates 112, 122 of jaw members 110, 120, respectively. As can be appreciated, the closure force imparted by jaw members 110, 120 determines the amount of compression of sealing plate engagement knobs 126c and, as a result, the gap distance "g" between sealing plates 112, 122.

With tissue grasped between sealing plates 112, 122 of jaw members 110, 120, respectively, electrosurgical energy may be supplied to one (or both) of tissue-sealing plates 112, 122 and through tissue to effect a tissue seal. Controlling the gap distance "g" between sealing plates 112 and 122 helps to ensure that an effective tissue seal is achieved. Once tissue has been sealed, a knife blade 182 (Figs. 6A-6C) may be advanced through knife channels 125, 126e of seal plate 122 and insulator 126, respectively, (and/or knife channel 115 of jaw member 110 (Figs. 6A-6C)) to divide tissue along the previously formed tissue seal.

With reference now to Figs. 5A-5C, a configuration and assembly of insulator 126 and sealing plate 122 onto jaw frame 124 of jaw member 120 is described

As best shown in Fig. 5A and as mentioned above, insulator 126 is configured for positioning atop jaw frame 124 and includes a pair of longitudinally-spaced jaw frame engagement knobs 126f extending therefrom, although greater or fewer than two jaw frame engagement knobs 126f may be provided. Jaw frame 124, on the other hand, includes a plurality of engagement apertures 124a defined therethrough, each of which is configured to receive one of the jaw frame engagement knobs 126f therein such that a free end 126g of each of the jaw frame engagement knobs 126f extends therefrom, as best shown in Fig. 5B . Engagement knobs 126f initially define a generally uniform, cylindrical configuration to facilitate passage through the generally circular engagement apertures 124a of jaw frame 124, although other complementary configurations may also be provided. Further, jaw frame 124 may include a recess or lip 124b defined about the periphery of each of the engagement apertures 124a on an underside surface 124c thereof, such that, as shown in Fig. 5B, free ends 126g of jaw frame engagement knobs 126f are positioned adjacent lips 124b of engagement apertures 124a when disposed through engagement apertures 124a.

With reference now to Figs. 5B and 5C, and initially to Fig. 5B, during assembly, insulator 126 is positioned atop jaw frame 124 such that jaw frame engagement knobs 126f of insulator 126 are disposed through engagement apertures 124a of jaw frame 124, with free ends 126g of jaw frame engagement knobs 126f extending therefrom. In order to secure insulator 126 about jaw frame 124, a heat-staking element (similar to heat-staking element 400 ( Fig. 4A ) is used to deform free ends 126g of jaw frame engagement knobs 126f of insulator 126 such that jaw frame engagement knobs 126f are inhibited from being translated back through engagement apertures 124a, similarly as discussed above with regard to insulator 126 and seal plate 122. More specifically, the heat-staking element is positioned about free ends 126g of each jaw frame engagement knob 126f and is heated to a sufficient temperature to deform jaw frame engagement knobs 126f of insulator 126, without substantially affecting jaw frame 124. As free ends 126g of jaw frame engagement knobs 126f are heated to achieve a state of deformability, free ends 126g of jaw frame engagement knobs 126f conform to lips 124b of jaw frame 124, which are disposed about engagement apertures 124a, as shown in Fig. 5C . Jaw frame engagement knobs 126f are then permitted to cool such that the free ends 126g thereof are retained within lips 124b of jaw frame 124. The heat-staking element (not shown) may define a working surface that is shaped complementary to the underside surface 124c of jaw frame 124 such that, in the deformed configuration, as shown in Fig. 5C , free ends 126g of jaw frame engagement knobs 126f are substantially flush with the underside surface 124c of jaw frame 124, i.e., such that free ends 126g of jaw frame engagement knobs 126f are completely disposed within lips 124b of jaw frame 124. As can be appreciated, in this deformed configuration, free ends 126g of jaw frame engagement knobs 126f are inhibited from passing back through engagement apertures 124a of jaw frame 124, thus securing insulator 126 atop jaw frame 124.

Referring now to Figs. 6A-6C, knife assembly 180 is shown in use in conjunction with end effector assembly 100. In use, as shown in Fig. 6A and as mentioned above, with jaw members 110, 120 disposed in the spaced-apart position, end effector assembly 100 is maneuvered into position such that tissue to be grasped, sealed, and or cut, is disposed between jaw members 110, 120. Next, moveable handle 40 (Fig. 1) is pulled proximally relative to fixed handle 50 (Fig. 1) such that jaw member 110 is pivoted relative to jaw member 120 from the spaced-apart position to the approximated position to grasp tissue therebetween (see Fig. 6B). Thereafter, electrosurgical energy may be supplied, e.g., via activation of switch 90 (Fig. 1), to tissue-sealing plate 112 and/or tissue-sealing plate 122 and conducted through tissue to effect a tissue seal, as discussed above. As shown in Fig. 6C, knife blade 182 may then be advanced from the retracted position (Fig. 6B) to the extended position (Fig. 6C), e.g., via activation of trigger 82 of trigger assembly 80 (Fig. 1), and through blade channels 115, 125 of jaw members 110, 120, respectively, to cut the previously sealed tissue grasped between jaw members 110, 120 (or to cut untreated tissue, depending on a particular purpose).

With continued reference to Figs. 6A-6C, knife assembly 180 includes a knife blade 182 defining a distal cutting edge 184 at the distal end 183 thereof. Knife blade 182 is coupled to a knife bar 186 at the proximal end 185 thereof. Knife bar 186 is selectively translatable, e.g., upon activation of trigger 82 (Fig. 1), through shaft 12 and relative to end effector assembly 100 to translate knife blade 182 from the retracted position (Fig. 6B) to the extended position (Fig. 6C).

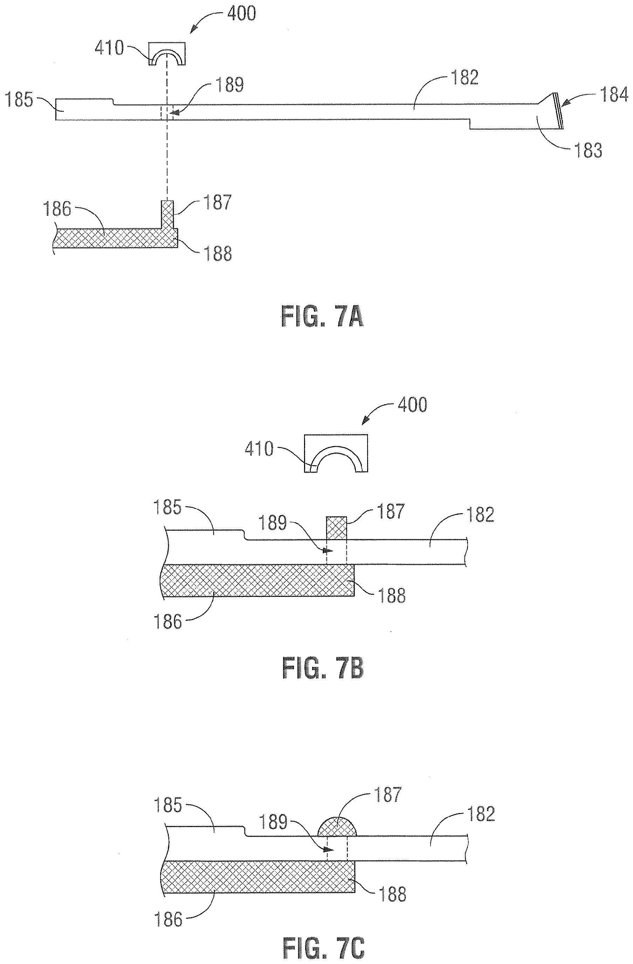

Turning now to Figs. 7A-7C, the configuration and assembly of knife assembly 180 in accordance with embodiments of the present invention will be described. As best shown in Fig. 7A, knife bar 186 includes an engagement knob 187 disposed at distal end 188 thereof. Engagement knob 187 is configured for insertion through a complementary-shaped engagement aperture 189 defined through knife blade 182 at proximal end 185 thereof. Similarly as described above, once engagement knob 187 has been inserted through engagement aperture 189, the free end of engagement knob 187 may be deformed via heat-staking without substantially affecting knife blade 182, e.g., using heat-staking element 400, to secure knife bar 186 and knife blade 182 to one another. Further, although knife blade 182 is shown positioned atop knife bar 186 and secured thereto via a single heat-staked knob/aperture engagement, heat-staking may alternatively be used to secure knife blade 182 and knife bar 186 in any other suitable position relative to one another and/or multiple heat-staked knob/aperture engagements may be provided.

From the foregoing and with reference to the various figure drawings, those skilled in the art will appreciate that certain modifications can also be made to the present disclosure without departing from the scope of the accompanying claims.

## Claims

1. A method of making a forceps, wherein the forceps comprises:
an end effector assembly (100) including first and second jaw members (110, 120), at least one of the jaw members (110, 120) movable relative to the other between a spaced-apart position and an approximated position for grasping tissue therebetween,
the method comprising the following steps in the manufacture of at least one of the jaw members (120):
providing an insulator (126) including at least one engagement knob (126c) extending therefrom, the at least one engagement knob (126c) having a diameter "d";
providing a tissue-sealing plate (122) having at least one engagement aperture (122b) defined therethrough, the tissue sealing plate (122) defining a tissue-sealing surface; and
positioning the tissue-sealing plate (122) about the insulator (126) such that the at least one engagement knob (126c) is inserted through the at least one engagement aperture (122b) and such that a free end (126d) of the at least one engagement knob (126c) extends through the at least one engagement aperture (122b) and extends from the tissue-sealing surface of the tissue-sealing plate (122),
**characterised in that** the method further comprises deforming the free end (126d) of the at least one engagement knob (126c) such that the free end (126d) of the at least one engagement knob (126c) is deformed from a first configuration having diameter "d" which is less than a diameter of the at least one engagement aperture (122b), to a second configuration having a diameter "D" which is greater than the diameter of the at least one engagement aperture (122b), thereby preventing the at least one engagement knob (126c) from passing through the at least one engagement aperture (122b) and securing the insulator (126) and the sealing plate (122) to one another,
wherein the step of deforming the free end (126d) of the at least one engagement knob (126c) is performed via heat-staking,
such that in the second configuration, the free end (126d) of the at least one engagement knob (126c) protrudes a pre-determined distance from the tissue-sealing surface to set a gap distance between the jaw members (110, 120) when the jaw members (110, 120) are disposed in the approximated position.

2. The method according to claim 1, wherein the gap distance is in the range of about 0.0254mm to about 0.1524mm

3. The method according to claim 1, wherein the at least one engagement knob (126c) is formed at least partially from a resiliently compressible material to set the gap distance between the jaw members (110, 120) in accordance with a closure pressure between the jaw members (110, 120).

## Patentansprüche

1. Verfahren zum Herstellen einer Zange, wobei die Zange Folgendes umfasst:
eine Endeffektoranordnung (100), die ein erstes und ein zweites Backenelement (110, 120) beinhaltet, wobei wenigstens eines der Backenelemente (110, 120) relativ zu dem anderen zwischen einer beabstandeten Position und einer angenäherten Position zum Erfassen von Gewebe dazwischen bewegbar ist,
wobei das Verfahren die folgenden Schritte bei der Fertigung von wenigstens einem der Backenelemente (120) umfasst:
Bereitstellen eines Isolators (126), der wenigstens einem Eingriffsknopf (126c) beinhaltet, der sich davon erstreckt, wobei der wenigstens eine Eingriffsknopf (126c) einen Durchmesser "d" aufweist;
Bereitstellen einer Gewebeversiegelungsplatte (122), die wenigstens eine Eingriffsöffnung (122b) aufweist, die dahindurch definiert ist, wobei die Gewebeversiegelungsplatte (122) eine Gewebeversiegelungsoberfläche definiert; und
Positionieren der Gewebeversiegelungsplatte (122) um den Isolator (126) herum derart, dass der wenigstens eine Eingriffsknopf (126c) durch die wenigstens eine Eingriffsöffnung (122b) hindurch eingeführt wird, und derart, dass ein freies Ende (126d) des wenigstens einen Eingriffsknopfs (126c) sich durch die wenigstens eine Eingriffsöffnung (122b) hindurch erstreckt und sich von der Gewebeversiegelungsoberfläche der Gewebeversiegelungsplatte (122) erstreckt, **dadurch gekennzeichnet, dass** das Verfahren ferner ein Verformen des freien Endes (126d) des wenigstens einen Eingriffsknopfs (126c) derart umfasst, dass das freie Ende (126d) des wenigstens einen Eingriffsknopfs (126c) aus einer ersten Konfiguration verformt wird, die einen Durchmesser "d" aufweist, der kleiner als ein Durchmesser der wenigstens einen Eingriffsöffnung (122b) ist, zu einer zweiten Konfiguration, die einen Durchmesser "D" aufweist, der über dem Durchmesser der wenigstens einen Eingriffsöffnung (122b) liegt, wobei dadurch verhindert wird, dass der wenigstens eine Eingriffsknopf (126c) durch die wenigstens eine Eingriffsöffnung (122b) hindurch verläuft und Befestigen des Isolators (126) und der Dichtungsplatte (122) zueinander,
wobei der Schritt des Verformens des freien Endes (126d) des wenigstens einen Eingriffsknopfs (126c) über ein Heißnieten durchgeführt wird,
derart, dass in der zweiten Konfiguration das freie Ende (126d) des wenigstens einen Eingriffsknopfs (126c) um einen zuvor bestimmten Abstand von der Gewebeversiegelungsoberfläche vorsteht, um einen Spaltabstand zwischen den Backenelementen (110, 120) einzustellen, wenn die Backenelemente (110, 120) in der angenäherten Position eingerichtet sind.

2. Verfahren nach Anspruch 1, wobei der Spaltabstand in dem Bereich von etwa 0,0254 mm bis etwa 0,1524 mm liegt.

3. Verfahren nach Anspruch 1, wobei der wenigstens eine Eingriffsknopf (126c) wenigstens teilweise aus einem elastisch komprimierbaren Material ausgebildet ist, um den Spaltabstand zwischen den Backenelementen (110, 120) gemäß einem Schließdruck zwischen der Backenelementen (110, 120) einzustellen.

## Revendications

1. Procédé de fabrication d'une pince, dans lequel la pince comprend :
un ensemble effecteur d'extrémité (100) comportant des premier et second éléments de mâchoire (110, 120), au moins l'un des éléments de mâchoire (110, 120) étant mobile par rapport à l'autre entre une position espacée et une position rapprochée pour saisir du tissu entre eux,
le procédé comprenant les étapes suivantes dans la fabrication d'au moins l'un des éléments de mâchoire (120) :
la fourniture d'un isolant (126) comportant au moins un bouton de mise en prise (126c) s'étendant à partir de celui-ci, l'au moins un bouton de mise en prise (126c) ayant un diamètre « d » ;
la fourniture d'une plaque de scellement de tissu (122) ayant au moins une ouverture de mise en prise (122b) définie à travers celle-ci, la plaque de scellement de tissu (122) définissant une surface de scellement de tissu ; et
le positionnement de la plaque de scellement de tissu (122) autour de l'isolant (126) de telle sorte que l'au moins un bouton de mise en prise (126c) est inséré à travers l'au moins une ouverture de mise en prise (122b) et de telle sorte qu'une extrémité libre (126d) de l'au moins un bouton de mise en prise (126c) s'étend à travers l'au moins une ouverture de mise en prise (122b) et s'étend à partir de la surface de scellement de tissu de la plaque de scellement de tissu (122), **caractérisé en ce que** le procédé comprend en outre la déformation de l'extrémité libre (126d) de l'au moins un bouton de mise en prise (126c) de telle sorte que l'extrémité libre (126d) de l'au moins un bouton de mise en prise (126c) est déformée d'une première configuration ayant un diamètre « d » qui est inférieur à un diamètre de l'au moins une ouverture de mise en prise (122b), à une seconde configuration ayant un diamètre « D » qui est supérieur au diamètre de l'au moins une ouverture de mise en prise (122b), permettant ainsi d'empêcher l'au moins un bouton de mise en prise (126c) de passer à travers l'au moins une ouverture de mise en prise (122b) et de fixer l'isolant (126) et la plaque de scellement (122) l'un à l'autre,
dans lequel l'étape de déformation de l'extrémité libre (126d) de l'au moins un bouton de mise en prise (126c) est effectuée par agrafage thermique,
de telle sorte que dans la seconde configuration, l'extrémité libre (126d) de l'au moins un bouton de mise en prise (126c) fait saillie d'une distance prédéterminée de la surface de scellement de tissu pour définir une distance d'espace entre les éléments de mâchoire (110, 120) lorsque les éléments de mâchoire (110, 120) sont disposés dans la position rapprochée.

2. Procédé selon la revendication 1, dans lequel la distance d'espace est comprise dans la plage d'environ 0,0254 mm à environ 0,1524 mm.

3. Procédé selon la revendication 1, dans lequel l'au moins un bouton de mise en prise (126c) est formé au moins partiellement à partir d'un matériau élastiquement compressible pour régler la distance d'espace entre les éléments de mâchoire (110, 120) conformément à une pression de fermeture entre les éléments de mâchoire (110, 120).
